# EUROPÄISCHE PATENTSCHRIFT

(11) **EP 0 373 321 B1**
(45) Veröffentlichungstag und Bekanntmachung des Hinweises auf die Patenterteilung: **08.06.1994**
(21) Anmeldenummer: 89119240.3
(22) Anmeldetag: 17.10.1989
(51) Int. Cl.: A61M 5/24, A61M 5/315

(54) **Wiederverwendbares Injektionsgerät zur Abgabe einer vorwählbaren Dosis**
Reusable injection device for the delivery of a preselected dose
Dispositif d'injection réntilisable pour administration d'un dose présélectionnable

(30) Priorität: 19.10.1988 CH 3892/88
(43) Veröffentlichungstag der Anmeldung: 20.06.1990
(73) Patentinhaber: Byk Gulden Lomberg Chemische Fabrik GmbH, D-78467 Konstanz (DE); Disetronic Medical Systems AG, 3401 Burgdorf (CH)
(72) Erfinder: Michel, Peter, Dr., CH-3400 Burgdorf (CH)

(56) Entgegenhaltungen:
- EP-A- 0 268 191
- WO-A-87/02895
- GB-A- 1 412 312

## Beschreibung

### Technisches Gebiet

Die Erfindung betrifft ein spritzenförmiges Injektionsgerät zur Abgabe einer vorwählbaren Dosis eines flüssigen Medikaments mit zwei miteinander kuppelbaren, hülsenförmigen Bauteilen, wovon einer als Halter für die Abgabemechanik, der andere als Ampullenhülse zur Aufnahme der Ampulle und der Kanüle ausgebildet ist, wobei die Abgabemechanik den Stopfen der Ampulle über eine Kolbenstange mit Kolbenstangen-Ansatz betätigen kann und eine Rückhaltfeder und einen Dosierknopf-Abschluß umfaßt.

### Stand der Technik

Ein Injektionsgerät dieser Art ist aus der WO 87/02895 bekannt. Dieses Gerät besteht aus zwei hülsenförmigen, miteinander verbindbaren Gehäuseteilen, wobei zum Gebrauch die Spritzampulle in das untere Gehäuseteil verschraubt wird. Die Dosis des abzugebenden Medikaments kann durch manuelle Einstellung vorgewählt werden.

Bei diesem und anderen ähnlich gebauten Geräten gemäß dem Stand der Technik besteht der Nachteil, daß sie nur für Medikamente eingesetzt werden können, bei denen eine mehrmalige Entnahme aus demselben Vorratsbehälter zulässig und meist auch gewünscht ist.

Bei der Therapie der funktionellen Erektionsstörung beim Mann wird das Medikament Papaverin ohne Konservierungsmittel in die Ampulle abgefüllt, weshalb aus Sterilitätsgründen nur eine einmalige Entnahme des Medikaments zugelassen werden kann.

Die Dosisbreite variiert je nach Krankheitsbild zwischen 0,25 - 2,0 ml, d.h. bei einer Entnahme von 0,25 ml werden die in der Ampulle verbleibenden 1,75 ml weggeworfen. Dieses Anforderungsprofil kann nun aber von den herkömmlichen Dosierhilfen (PEN-Injektionsgeräte) nicht erfüllt werden.

### Beschreibung der Erfindung

Hier will die Erfindung Abhilfe schaffen. Der Erfindung liegt die Aufgabe zugrunde, ein Injektionsgerät zu schaffen, welches vom Arzt auf eine feste, einmalige Dosisabgabe vorprogrammiert werden kann, die vom Patienten nicht mehr verändert werden kann.

Die Erfindung löst die gestellte Aufgabe mit einem Injektionsgerät, bei dem die Abgabemechanik derart ausgebildet ist, daß nur eine einzige Dosisabgabe aus der gleichen Ampulle erfolgen kann.

Die durch die Erfindung erreichten Vorteile sind im wesentlichen darin zu sehen, daß dank des erfindungsgemäßen Injektionsgerätes höchste Bedienungssicherheit gewährleistet ist, da weder eine beabsichtigte noch eine ungewollte Zweitentnahme des Medikaments möglich ist.

Gegenstand der Erfindung ist ein spritzenförmiges Injektionsgerät zur Abgabe einer vorwählbaren Dosis eines flüssigen Medikaments mit zwei miteinander kuppelbaren, hülsenförmigen Bauteilen, wovon einer als Halter für die Abgabemechanik, der andere als Ampullenhülse zur Aufnahme der Ampulle und der Kanüle ausgebildet ist, wobei die Abgabemechanik den Stopfen der Ampulle über eine Kolbenstange mit Kolbenstangen-Ansatz betätigen kann und eine Rückhaltfeder und einen Dosierknopf-Abschluß umfaßt, dadurch gekennzeichnet, daß die Abgabemechanik mittels eines Verschlußdeckels mit dem Mechanikhalter unlösbar verbindbar ist, die Kolbenstange aus einem inneren den Kolbenstangen-Ansatz tragenden Hohlzylinder und einem mit diesem inneren Hohlzylinder einstückig ausgebildeten äußeren Hohlzylinder besteht, der an seiner äußeren Mantelfläche einen Führungsnocken aufweist, der hülsenförmige Mechanikhalter an seiner Innenseite mit Längsschlitzen unterschiedlicher Länge versehen ist, in welchen ein entsprechender Führungsnocken der Kolbenstange gleiten kann, und ein nur mit einem Spezialschlüssel lösbarer Verriegelungsring vorgesehen ist, der die Abgabemechanik so verriegelt, daß der Führungsnocken sich nur in einem vorwählbaren Längsschlitz bewegt.

Weitere vorteilhafte Ausgestaltungen der Erfindung ergeben sich aus den Unteransprüchen.

Ein Ausführungsbeispiel der Erfindung, welches zugleich das Funktionsprinzip erläutert, ist in der Zeichnung dargestellt und wird im folgenden näher beschrieben.
- Fig. 1: stellt einen Achsenlängsschnitt durch das Injektionsgerät gemäß der Erfindung dar.
- Fig. 2: zeigt einen Querschnitt längs der Linie II-II in Fig. 1.
- Fig. 3: zeigt einen Querschnitt längs der Linie III-III in Fig. 1.
- Fig. 4: zeigt eine perspektivische Ansicht des Injektionsgerätes gemäß der Erfindung.

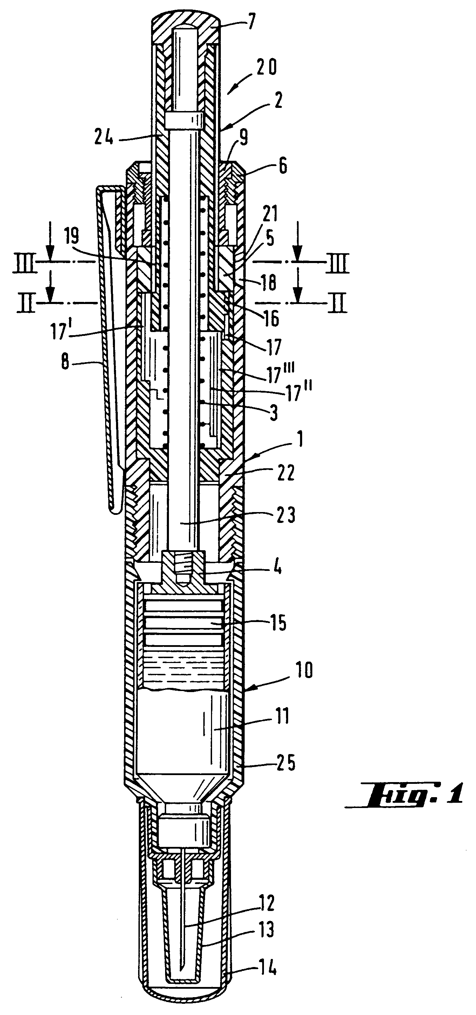

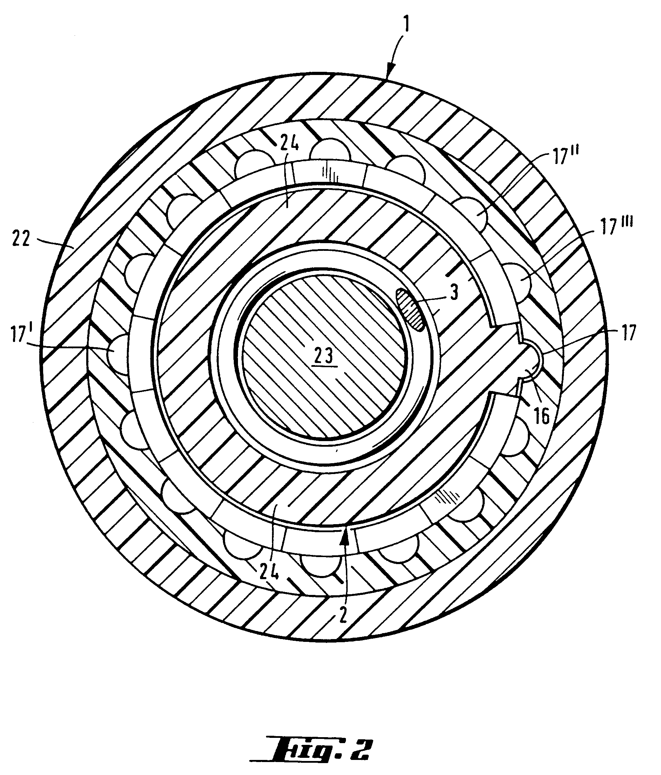

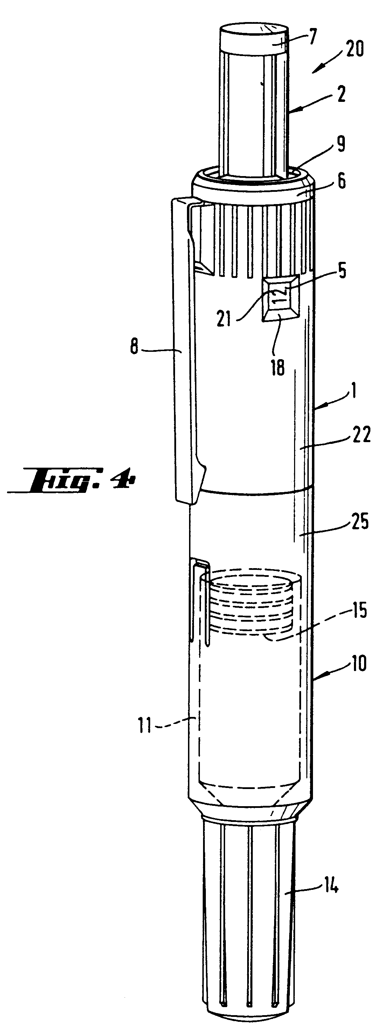

Das in Fig. 1 im Längsschnitt dargestellte erfindungsgemäße Injektionsgerät besteht im wesentlichen aus zwei miteinander kuppelbaren, hülsenförmigen Bauteilen 1,10, wovon einer als Halter 1 für die Abgabemechanik 20, der andere als Ampullenhülse 10 zur Aufnahme der Ampulle 11 und der Kanüle 12 ausgebildet ist. Das zusammengesetzte und mittels des Verschlußdeckels 6 gesicherte Injektionsgerät kann mittels des Klips 8 wie ein Füllfederhalter getragen werden (Fig. 4). Die Abgabemechanik 20 besteht aus einer zylindrischen Kolbenstange 2 mit einem inneren, einen Kolbenstangen-Ansatz 4 tragenden Hohlzylinder 23 und einem, mit diesem inneren Hohlzylinder 23 einstückig ausgebildeten äußeren Hohlzylinder 24, der an seiner äußeren Mantelfläche einen Führungsnocken 16 aufweist (Fig. 2). Gegen außen ist die Abgabemechanik als manuell betätigbarer Dosierknopf 7 ausgebildet. Die Abgabemechanik 20 wirkt bei zusammengesetztem Injektionsgerät mittels des Kolbenstangen-Ansatzes 4 auf den Boden des Ampullenstopfens 15 der Medikamentenampulle 11.

Die Ampullenhülse 10 besteht aus einem die Ampulle 11 aufnehmenden Gehäuse 25, welches mit einer nicht dargestellten Kupplung fest mit dem Gehäuse 22 des Mechanikhalters 1 verbunden werden kann. Die mit der Ampulle 11 kommunizierende Kanüle 12 ist mittels der inneren Schutzkappe 13 und der äußeren Verpackungshülse 14 gegen Beschädigung gesichert.

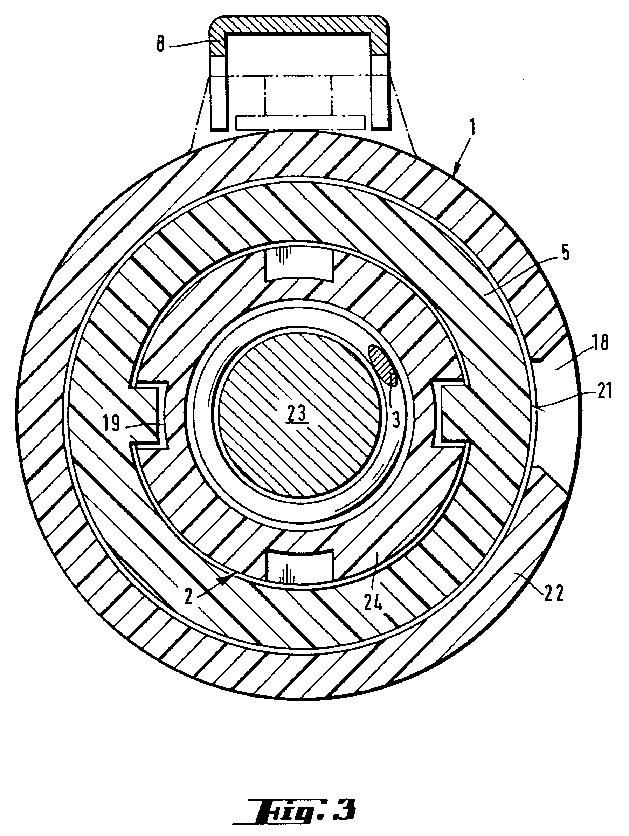

Die weitere detaillierte Beschreibung, insbesondere der Abgabemechanik 20 anhand der Schnittdarstellung gemäß Fig. 2 und 3 soll gleichzeitig die Funktion des erfindungsgemäßen Injektionsgerätes erläutern.

Auf dem äußeren Hohlzylinder 24 ist mittels Nocken-Nut-Kupplungen 19 ein Dosierring 5 radial fest angebracht, so daß sich dieser bei einer Rotation der Abgabemechanik 20 mitdreht. Eine am äußeren Umfang des Dosierringes 5 angebrachte Skalierung 21 kann durch ein im Gehäuse 22 des Mechanikhalters 1 eingelassenes Sichtfenster 18 abgelesen werden (Fig. 3).

Die Abgabemechanik 20 ist mittels der Rückhaltefeder 3 längsbeweglich im entsprechend als Hohlzylinder ausgebildeten Mechanikhalter 1 angeordnet und durch den mit dem Gehäuse 22 des Mechanikhalters 1 verschraubbaren Verschlußdeckel 6 gesichert. In Fig. 1 ist die Ruheposition der Abgabemechanik 20 dargestellt, bei welcher der Dosierring 5 mit der Kraft der auf die Abgabemechanik wirkenden Rückhaltefeder 3 gegen den eingeschraubten Verschlußdeckels 6 anliegt.

Das Gehäuse 22 des Mechanikhalters 1 ist auf seiner Innenseite mit Längsschlitzen 17 unterschliedlicher Länge versehen, in welche der entsprechende Führungsnocken 16 der Abgabemechanik 20 gleiten kann.

Der Hub der Abgabemechanik 20 und damit die ausstoßbare Medikamentendosis wird somit von der Länge des mit dem Führungsnocken 16 zusammenwirkenden Längsschlitzes 17 bestimmt. Gleitet der Führungsnocken 16 in einem kurzen Längsschlitz 17, wie in Fig. 1 dargestellt, so ist die Medikamentendosis gering, gleitet der Führungsnocken 16 in einem längeren Längsschlitz 17''', so ist die Medikamentendosis größer.

Die Anpassung der Medikamentendosis an die Bedürfnisse des Patienten erfolgt ausschließlich durch den Arzt. Dieser kann den Verriegelungsring 9 des Gerätes mittels eines nicht dargestellten Spezialschlüssels lösen und soweit nach hinten zurückschrauben, bis der Führungsnocken 16 der Abgabemechanik 20 aus dem jeweiligen Längsschlitz 17 ausrastet. Nun kann der Arzt durch Rotation der ausgerasteten Abgabemechanik 20 und erneutes Einrasten des Führungsnockens 16 in einen kürzeren oder längeren Längsschlitz 17 die gewünschte Medikamentendosis auswählen. Die Skalierung 21 am Dosierring 5 zeigt dabei am Sichtfenster 18 die gewählte Einstellung an. Wird nun der Verriegelungsring 9 vom Arzt mittels des Spezialschlüssels wieder festgezogen, so kann sich der Führungsnocken 16 nur im ausgewählten Längsschlitz 17 in Längsrichtung bewegen. Die ausstoßbare Medikamentendosis ist einzig durch die Tiefe des mit dem Führungsnocken 16 zusammenwirkenden Längsschlitzes 17 definiert.

Wird das erfindungsgemäße Injektionsgerät bestimmungsgemäß verwendet, so kann keine weitere (zweite) Dosis der Ampulle 11 entnommen werden, obwohl möglicherweise noch ein genügender Medikamentenvorrat in der Ampulle 11 vorhanden ist. Die Abgabemechanik 20 ist somit für eine Einmalentnahme mit fester Dosierung eingestellt und kann vom Patienten nicht geändert werden.

Um eine weitere Medikamentendosis zu spritzen, muß der Patient die Ampullenhülse 10 vom Mechanikhalter 1 lösen und zusammen mit der Ampulle 11 wegwerfen. Nur durch Einsetzen einer frischen Ampulle 11 kann das Gerät wieder verwendet werden.

Damit der Patient nicht in Versuchung kommt, eine allfällige Restmenge des Medikaments mittels irgendwelcher Manipulationen aus der Ampulle 11 zu entnehmen und wieder zu verwenden, wird das die Ampulle 11 aufnehmende Gehäuse 25 so ausgebildet, daß die Ampulle 11 nur sehr schwer daraus entfernt werden kann. Überdies wird das Gehäuse 25 vorzugsweise aus einem optisch undurchsichtigen Material, beispielsweise Polyamid, hergestellt, so daß eine allfällige Restmenge nicht sichtbar ist.

Um eine neue Dosis Medikament applizieren zu können, kann dann lediglich der alte untere hülsenförmige Bauteil 10 durch einen neuen ersetzt werden. Der Patient kann auf diese Weise nicht mehr feststellen, ob und allenfalls welche Restmenge Medikament in der Ampulle 11 zurückbleibt. Dies ist besonders wichtig beim unkonserviert zum Einsatz kommenden Papaverin, welches im Falle einer Überdosierung zu irreparablen Schäden führen würde.

## Patentansprüche

1. Spritzenförmiges Injektionsgerät zur Abgabe einer vorwählbaren Dosis eines flüssigen Medikaments mit zwei miteinander kuppelbaren, hülsenförmigen Bauteilen (1,10), wovon einer als Halter (1) für die Abgabemechanik (20), der andere als Ampullenhülse (10) zur Aufnahme der Ampulle (11) und der Kanüle (12) ausgebildet ist, wobei die Abgabemechanik (20) den Stopfen (15) der Ampulle (11) über eine Kolbenstange (2) mit Kolbenstangen-Ansatz (4) betätigen kann und eine Rückhaltfeder (3) und einen Dosierknopf-Abschluß (7) umfaßt, dadurch gekennzeichnet, daß die Abgabemechanik (20) mittels eines Verschlußdeckels (6) mit dem Mechanikhalter (1) unlösbar verbindbar ist, die Kolbenstange (2) aus einem inneren den Kolbenstangen-Ansatz (4) tragenden Hohlzylinder (23) und einem mit diesem inneren Hohlzylinder (23) einstückig ausgebildeten äußeren Hohlzylinder (24) besteht, der an seiner äußeren Mantelfläche einen Führungsnocken (16) aufweist, der hülsenförmige Mechanikhalter (1) an seiner Innenseite mit Längsschlitzen (17) unterschiedlicher Länge versehen ist, in welchen ein entsprechender Führungsnocken (16) der Kolbenstange (2) gleiten kann, und ein nur mit einem Spezialschlüssel lösbarer Verriegelungsring (9) vorgesehen ist, der die Abgabemechanik (20) so verriegelt, daß der Führungsnocken (16) sich nur in einem vorwahlbaren Längsschlitz (17) bewegt.

2. Injektionsgerät nach Anspruch 1, dadurch gekennzeichnet, daß die Längsschlitze (17) in gleichmäßigen Abständen und vorzugsweise mit zunehmender Länge am Umfang des Innenmantels des Gehäuses (22) des Mechanikhalters (1) angeordnet sind.

3. Injektionsgerät nach einem der Ansprüche 1 bis 2, dadurch gekennzeichnet, daß ein Dosierring (5) vorzugsweise mittels Nocken-Nut-Kupplungen (19) radial fest mit der Kolbenstange (2) verbunden ist und an seinem äußeren Umfang eine Skalierung (21) aufweist, die durch ein Sichtfenster (18) im Gehäuse (22) des Mechanikhalters (1) von außen sichtbar ist.

4. Injektionsgerät nach einem der Ansprüche 1 bis 3, dadurch gekennzeichnet, daß die Ampulle (11) schwer lösbar mit der Ampullenhülse (10) verbunden ist.

5. Injektionsgerät nach einem der Ansprüche 1 bis 4, dadurch gekennzeichnet, daß das Gehäuse (25) aus einem optisch undurchsichtigen Material, beispielsweise Polyamid, besteht.

6. Injektionsgerät nach einem der Ansprüche 1 bis 5, dadurch gekennzeichnet, daß das die Ampulle (11) aufnehmende Gehäuse (25) einstückig ausgebildet ist.

## Claims

1. Syringe-shaped injection device for dispensing a preset dose of a liquid drug with two sleeve-like components (1, 10) which can be connected together, one of which is designed as the holder (1) for the dispensing mechanism (20) and the other as the ampoule sleeve (10) to accommodate the ampoule (11) and the cannula (12), where the dispensing mechanism (20) can actuate the plug (15) of the ampoule (11) by a piston rod (2) with a piston rod neck (4) and comprises a retaining spring (3) and a dosing button cap (7), characterized therein that the dispensing mechanism (20) is connected by means of a cap (6) with the mechanism holder (1) such that it cannot be disengaged, the piston rod (2) consists of an inner hollow cylinder (23) bearing the piston rod neck (4) and, formed integrally with this inner hollow cylinder (23), of an outer hollow cylinder (24) which has a cam follower (16) on its outer sleeve surface, the interior of the sleeve-like mechanism holder (1) is provided with longitudinal slots (17) of varying length in which a corresponding cam follower (16) of the piston rod (2) can slide and a locking ring (9), which only can be released by means of a special key, locking the dispensing mechanism (20) in a manner such that the cam follower (16) can only move in a preselected longitudinal slot (17).

2. Syringe device according to claim 1 characterized therein that the longitudinal slots (17) are configured equidistantly and preferably with increasing length around the periphery of the inner sleeve of the housing (22) of the machanism holder (1).

3. Syringe device according to any one of claims 1 to 2, characterized therein that the dose ring (5) is firmly connected radially to the piston rod (2) preferably by means of cam/slot couplings (19) and is provided on its outer periphery with a scale (21) which is visible from outside through a window (18) in the housing (22) of the mechanism holder (1).

4. Syringe device according to any one of claims 1 to 3, characterized therein that the ampoule (11) is connected to the ampoule sleeve (10) such that it is difficult to disengage.

5. Syringe device according to any one of claims 1 to 4, characterized therein that the housing (25) is made of an optically opaque material, for example polyamide.

6. Syringe device according to any one of claims 1 to 5, characterized therein that the housing (25) accommodating the ampoule (11) is designed as a single component.

## Revendications

1. Instrument d'injection pour l'administration d'une dose pouvant être présélectionnée d'un médicament liquide, comportant deux composants (1, 10) en forme de manchons, pouvant être accouplés l'un à l'autre, dont l'un est configuré comme support (1) pour le mécanisme d'administration (20), l'autre étant configuré sous forme de manchon d'ampoule (10) pour la réception de l'ampoule (11) et de la canule (12), le mécanisme d'administration (20) pouvant actionner le bouchon (15) de l'ampoule (11) par l'intermédiaire d'une tige de piston (2) comportant une garniture (4) de tige de piston, un ressort de retenue (3) et une fermeture (7) de bouton doseur, caractérisé en ce que le mécanisme d'administration (20) peut être relié de manière inamovible au support de mécanisme (1) au moyen d'un couvercle de fermeture (6), en ce que la tige de piston (2) est constituée d'un cylindre intérieur creux (23) portant la garniture (4) de tige de piston et d'un cylindre extérieur creux (24) réalisé d'une seule pièce avec ce cylindre intérieur creux (23), le cylindre extérieur creux (24) présentant sur sa surface d'enveloppe extérieure une saillie de guidage (16), en ce que le support de mécanisme (1) en forme de manchon est doté sur sa face intérieure de fentes longitudinales (17) de différentes longueurs, dans lesquelles une saillie correspondante de guidage (16) de la tige de piston (2) peut glisser, et en ce qu'est prévue une bague de verrouillage (9) qui ne peut être libérée qu'avec une clé spéciale, la bague de verrouillage (9) verrouillant le mécanisme d'administration (20) de telle sorte que la saillie de guidage (16) ne se déplace que dans une fente longitudinale (17) présélectionnable.

2. Instrument d'injection selon la revendication 1, caractérisé en ce que les fentes longitudinales (17) sont disposées à des distances égales, de préférence dans un ordre de longueur croissante, à la périphérie de l'enveloppe intérieure du boîtier (22) du support de mécanisme (1).

3. Instrument d'injection selon l'une des revendications 1 et 2, caractérisé en ce qu'une bague doseuse (5) est solidarisée radialement avec la tige de piston (2), de préférence au moyen d'accouplements (19) à saillies et rainures, et présente à sa périphérie extérieure une échelle (21) qui est visible de l'extérieur à travers une fenêtre d'observation (18) pratiquée dans le boîtier (22) du support de mécanisme (1).

4. Instrument d'injection selon l'une des revendications 1 à 3, caractérisé en ce que l'ampoule (11) est reliée au manchon d'ampoule (10) de manière à être difficilement libérée.

5. Instrument d'injection selon l'une des revendications 1 à 4, caractérisé en ce que le boîtier (25) est réalisé en un matériau opaque, par exemple du polyamide.

6. Instrument d'injection selon l'une des revendications 1 à 5, caractérisé en ce que le boîtier (25) reprenant l'ampoule (11) est réalisé en une seule pièce.
